# EUROPEAN PATENT APPLICATION

(11) **EP 0 791 660 A1**
(43) Date of publication of application: **27.08.1997**
(21) Application number: 97300988.9
(22) Date of filing: 14.02.1997
(51) Int. Cl.: C12Q 1/68

(54) **A novel diagnostic marker for splicing variants of genes associated with neurological function**

(30) Priority: 22.02.1996 US 12077
(71) Applicant: SMITHKLINE BEECHAM CORPORATION, Philadelphia Pennsylvania 19103 (US); WASHINGTON UNIVERSITY, St. Louis, Missouri 63110 (US); University of South Florida, Tampa, Florida 33613 (US)
(72) Inventor: Hardy, John, Mayo Clinic Jacksonville, Jacksonville, FL 32224 (US); Goate, Alison, Washington University, St. Louis, Missouri 63110 (US); Barton, Amanda, SmithKline Beecham Pharmaceuticals, Harlow, Essex CM19 5AW (GB)
(74) Representative: Valentine, Jill Barbara

(57) **Abstract**

Methods are described for detecting the presence or absence of a four amino acid motif (VRXQ) in expressed proteins that arise from aberrant alternative splicing of premRNA in genes associated with normal neurological function which are useful for detecting neurodegenerative disease. The presence of these variants suggest that mutational events in these genes have occurred. Methods to measure the levels of gene expression of such genes to detect neurodegenerative disease are provided. Nucleotide sequences and intron-exon junctional sequences of examples of this splicing variant and probes for detecting this variant which are useful as diagnostic reagents are also provided.

## Description

### BACKGROUND OF THE INVENTION

In eukaryotes, the initial transcription of genomic DNA into RNA proceeds in the nucleus and yields a contiguous full-length reverse complementary heteronuclear RNA (hnRNA) primary transcript. The hnRNA contains regions or contiguous blocks of nucleotide sequence that end up in the final mRNA (exons) interspersed between "intervening" nucleotide sequences (introns) that do not. In addition to adenylyl methylation and polyadenylation, these hnRNAs are extensively modified in a process referred to as RNA "splicing" wherein discontiguous exons are joined and the intervening intron precisely deleted as an RNA "lariat" from the final mature mRNA transcript (B. Rushkin et al. Cell 1984, 38:317; R.A. Padgett et al. Science 1984, 225:898). RNA splicing is a complex process involving large protein-RNA assemblies called spliceosomes that coordinate the concerted excision and ligation events to yield intron-free mRNAs (M.M. Konarska and P.A. Sharp Cell 1987, 49:763; R. Reid et al. Cell 1988, 53:949; T.A. Steitz Sci. Am. 1988, 258:56).

In normal RNA processing, the resultant mRNA reflects the linear sequence orientation of the exons in the hnRNA; however all exons do not end up in the final transcripts. Rather, several of the resultant mRNAs have only certain exons that result from "alternatively spliced" hnRNA, wherein discontiguous intron-exon junctions are spliced to bring for instance exon1 and exon 4 into juxtaposition rather than exon1 and exon 2. Therefore, several mRNAs may arise from one gene sequence or hnRNA. Not all possible combinations of exons are normally represented in actual mRNA pools arising from one hnRNA as determined by mRNA, cDNA and protein analyses. As an example with three exons (Figure 1), while seven combinations are possible (exon1-exon2-exon3, exon1-exon2, exon1-exon3, exon2-exon3, exon1, exon2, or exon3) perhaps only two (exon1-exon2-exon3 and exon1-exon3) may actually result and be expressed at any appreciable level. These alternatively spliced transcripts are sometimes referred to as "variants". However, for purposes of this invention splice "variant" refers to heretofore unrepresented or expressed mRNAs arising from potential alternative splice sites that result from genomic mutation altering the structure of the hnRNA so that these splices now occur.

The location of splice sites in an hnRNA primary transcript can be determined by comparing the sequences of the corresponding genomic DNA with that of cDNA prepared by copying the corresponding mature mRNA. Any discontinuities between the genomic DNA and cDNA sequences mark the exon-intron boundaries. Such analyses of a number of different RNAs have defined moderately -short "consensus" sequences at the intron-exon boundaries in pre-mRNA and a tendency for a pyrimidine-rich region just upstream of the 3' splice junction (Figure 2). The only universally conserved nucleotides are the first two (GU) and last two (AG) in the intron (Figure 2), though there is a propensity for AG at the 5' exon termini and an initial G at the 3' exon. Only 30-40 nucleotides in the center portion of introns are necessary for efficient splicing. There is also a conserved A within the context of the pyrimidine rich region of the intron (Figure 2) (..PyrPyrPurAPyrnAG; where Pyr is a pyrimidine and Pur is a purine nucleotide) which is the branch point where the cleaved 5' exon-intron junction loops back to form the "lariat" splicing intermediate (Padgett et al. Science 1984, 225:898). Genetic point mutations that delete or alter these conserved intronic nucleotides (5' GU, 3' AG, or branch point A) would eliminate these splice junctions and prevent normal splicing yielding aberrantly truncated transcripts or transcripts where this exon is deleted and another downstream exon spliced in, that normally may not be spliced in.

A final mechanism for splice variation occurs when several GU or AG dinucleotide motifs occur near consensus intron splice regions of 5' exon-intron or 3' intron-exon boundaries, respectively, such that the splicing system may sometimes not correctly distinguish the correct splice site resulting in alternate protein product some of which may be non-functional or aberrant.

Multiple examples of splice variations exist, many of which are associated with diseases or related disorders. Previous genetic linkage studies have shown a G to A mutation at the 3' splice junction of exon 8 of the gene encoding lysosomal acid lipase. Defects in this gene are associated with cholesterol ester storage disease that result in premature artherosclerosis, hepatomegaly, and elevated LDL cholesterol (U. Seedorf et al. Arterioscler. Throb. Vasc. Biol. 1995, 15: 773-778). Two mutations at the exon 1/intron 1 boundary altered the hepatic specific splicing of the human hydroxymethylbilane synthase gene (third enzyme in heme biosynthetic pathway) and resulted in an enzyme with half-normal activity (K.H. Astrin Human Mutat. 1994, 4:243-252). Deficiency of this enzyme activity eventually results in acute intermittent porphyria (AIP), an autosomal dominant inborn error of metabolism in which life-threatening attacks are precipitated by ecogenetic factors. Molecular cloning of cDNA and genomic DNA have provided probes allowing presymptomatic detection of these gene defects. In Menke's disease, a point mutation at the - 2 exonic position of a splice donor site in the middle of the gene causes exon-skipping and activation of a cryptic splice acceptor site (S.G. Kaler et al. Nat. Genet. 1994, 8:195-202). Exon skipping of the entire exon 19 results from a G to A point mutation at the 5' donor site of intron 19 in muscle phosphofructokinase deficiency (T. Hamaguchi Biochem. Biophys. Res. Comm. 1994, 202:444-449). Aberrant RNA splicing from a splice site mutant in the interleukin-2 receptor gamma (gIL2-R) gene results in the generation of an abundant non-functional gIL2-R containing a small intronic insertion and a second mutant form with 5-fold lower affinity (J.P. DiSanto et al. Proc. Natl. Acad. Sci. 1994, 91:9466-9470). These isoforms produce an atypical form of an X chromosome-linked severe combined immunodeficiency disease.

The presence of splice variants can be used as diagnostic markers of diseases associated with genetic mutations. For example, the expression of the exon 6 splice variant (v6) of the cell adhesion molecule CD44 is correlated with the expression of the tumor suppressor gene p53. Both have been shown to be markers of tumor progression in colorectal cancer (J.W. Mulder et al. Gut 1995, 36:76-80; Y. Matsumura Lancet 1992, 340:1053-1058). Asymptomatic carriers of the acute intermittent porphyria were identified by identification of a mutant allele containing a CG to CT transversion at the exon1/intron 1 boundary via in vitro amplification of DNA followed by hybridization of the target sequence to allele-specific oligonucleotides.

Accordingly, splicing variants have been observed in several gene loci and several diseases. Identification of these variants has proven to be especially useful in diagnosis and detection of asymptomatic carriers.

### SUMMARY OF THE INVENTION

A novel insertional motif that arises from splice mutations or alternative utilization of cryptic or less preferred splice donor sites has now been identified. These splicing variations result in the in-frame insertion within a normal protein sequence of four amino acids, valine-arginine-X-glutamine (VRXQ), where X is a hydrophilic amino acid. This motif has been identified in splice variants of a receptor, an enzyme, and a putative channel protein, all of which are involved in normal neurological functioning. Identification of this motif allows for screening of genes and gene products for splice variations.

A method for the detection of this motif in expressed proteins in vitro or in situ with the use of specific antisera, polyclonal or monoclonal antibodies is provided. A method for the detection of allele-specific genetic mutations using selected oligonucleotides with standard hybridization-based detection techniques is also provided. A method for diagnosing Alzheimer's Disease (AD) by detecting differences in levels of transcripts having the VRXQ insertion or proteins encoded therefrom is further provided. A preferred embodiment of such method for detecting AD provides for the detection of Familial Adult Onset Alzheimer's Disease (FAD).

### BRIEF DESCRIPTION OF THE FIGURES

Figure 1 is a schematic of potential alternative splicing with 3 exons and 4 introns.

Figure 2 is a schematic of the consensus exon-intron-exon structure and sequence.

Figure 3 provides the sequence of the VRSQ variant of the presenilin 1 gene. SEQ ID NOS: 1 - 2

Figure 4 provides PS-1 Oligonucleotide Probes. SEQ ID NOS: 3 - 5

Figure 5 provides tabulated results of quantification of the ISH signal for PS-1-long and PS-1-short mRNAs in human brain.

### DETAILED DESCRIPTION OF THE INVENTION

The presenilin 1 (herein "PS-1") gene encodes a neuropeptide predicted to be a classical seven transmembrane protein (Sherrington et al. Nature 1995, 375:754-760). Missense mutations within this gene have been found in several families exhibiting early-onset Alzheimer's disease. Genomic analysis has revealed the intron-exon boundaries of the hnRNA. A common polymorphism located within the intron 3' to exon 9 was identified in early onset AD patients. This polymorphism also showed a strong association with the occurrence of typical late onset AD families. This particular mutation did not produce an alteration in the coding sequence but is typical of variations leading to alternatively spliced proteins.

Other mutations within different introns of the PS-1 gene have been identified. These lead to alternatively spliced variants as well. One novel variant of the PS-1 protein isolated from a human cerebellar cDNA library contains a four amino acid insertion between codons 26 and 27 (VRSQ) (Figure 3). This variant arises from alternative use of a 5' exon donor site in the exon 3/intron 3 boundary and results in the loss of some potential phosphorylation sites. A similar motif (VRXQ- where X is a hydrophilic amino acid) arising from aberrant splicing has also arisen due to alternative splicing in several other neurological proteins as well.

For example, the mRNA for tyrosine hydroxylase, the rate limiting enzyme in the synthesis of catecholomines, can undergo alternative splicing to produce several different isoforms (Kobayashi et al. J. Biochem. 1988, 103(6) 907-12; Lewis et al. Neuroscience 1993, 54(2) 477-92). The identified variants contain a 12 bp insertion encoding the sequence VRGQ. Isoforms containing the VRGQ insertion have also been found to exhibit alterations in phosphorylation by MAP kinase (Sutherland et al. Eur J Biochem. 1993, 217(2) 715-22). Furthermore, a tyrosine hydroxylase variant containing this insertion has been implicated in Parkinson's disease.

Another neuropeptide, gamma-Aminobutyric acid A (GABAA) receptor, undergoes alternative splicing to yield a multiplicity of transcripts (Whiting et al. P.N.A.S. 1990, 87(24) 9966-70; Lasham et al. Biochem. Soc. Trans. 1991, 19(1) 9S). GABA receptors are multisubunit ligand gated ion channels which mediate neuronal inhibition by GABAA and are composed of at least four subunit types (alpha, beta, gamma, and delta). The beta 4 subunit can undergo alternative splicing at two 5'-donor splice sites separated by 12 bp in the region that encodes the presumed intracellular loop between transmembrane domains M3 and M4. The insertion of the 12 bp sequence results in the addition of a VREQ motif (Bateson et al. J. Neurochem 1991, 56(4) 1437-40).

In all three neurological proteins, the alternative splice site generates variants containing a specific motif (VRXQ) which appears to be intracellularly located and alters phosphorylation by various kinases.

In the present invention, a method for detecting the presence of the VRXQ motif in polyadenylated messenger RNA transcripts (polyA mRNA) and resultant expressed proteins, (where V is valine, R is arginine, X is any hydrophilic amino acid residue, and Q is glutamine) or in cDNA resulting from these RNAs is provided. A method for quantitating such transcripts encoding and proteins having a VRXQ motif are also provided. Oligonucleotides having the anticodon sequences associated with the VRXQ motif having degenerate positions at the third base position of each codon can be used for the detection and quantitation of mRNA. Additionally, these oligonucleotides can be associated with codon sequences and used for the detection of cDNAs, and quantitation of the transcript from which the cDNA was derived. For example, codon and anticodon oligonucleotides for VRNQ comprise GU(N) AG(A/G) AA(C/U) CA(A/G) and the reverse complement. Hybridization of appropriate oligonucleotides can be detected and quantitated directly by procedures well known to those of skill in the art using radioactively or fluorescently labeled oligonucleotides. Indirect detection and quantitation procedures such as, but not limited to, biotinylated oligonucleotides/strepavidin-horseradish peroxidase, enhanced chemiluminescent detection, or fluorescently tagged strepavidins can also be performed.

Specific antibodies against the VRXQ motif can also be used for detection of the motif and quantitation of proteins having the motif. Various procedures known in the art may be used for the production of such antibodies.

For example, these antibodies can be obtained by direct injection of a polypeptide containing a VRXQ motif into an animal, preferably a nonhuman. The antibody so obtained will then bind to polypeptides containing this motif. Such antibodies can then be used to isolate and quantitate polypeptides containing this motif from tissues.

For preparation of monoclonal antibodies, any technique which provides antibodies produced by continuous cell line cultures can be used. Examples include the hybridoma technique (Kohler and Milstein, Nature 1975, 256:495-497), the trioma technique, the human B-cell hybridoma technique (Kozbor et al., Immunology Today 1983, 4:72), and the EBV-hybridoma technique to produce human monoclonal antibodies (Cole et al. in Monoclonal Antibodies and Cancer Therapy, Alan R. Liss, Inc., 1985, pp. 77-96).

Techniques described for the production of single chain antibodies (U.S. Patent 4,946,778) can be adapted to produce single chain antibodies to the immunogenic motif of this invention. Also, transgenic mice may be used to express humanized antibodies to polypeptides containing this motif.

Primary antibody-antigen reactions can be visualized and quantitated secondarily by standard enzyme-linked immunosorbent assay (ELISA) procedures. An ELISA assay initially comprises preparing an antibody specific to a VRXQ motif, preferably a monoclonal antibody. In addition a reporter antibody is prepared against the monoclonal antibody. To the reporter antibody is attached a detectable reagent such as horse radish peroxidase. A sample is then removed from a host and incubated on a solid support, e.g., a polystyrene dish, that binds the proteins in the sample. Any free protein binding sites on the dish are then covered by incubating with a non-specific protein like BSA. Next, the monoclonal antibody is incubated in the dish during which time the monoclonal antibodies attach to any proteins containing the VRXQ motif attached to the polystyrene dish. All unbound monoclonal antibody is washed out with buffer. The reporter antibody linked to horseradish peroxidase is then placed in the dish resulting in binding of the reporter antibody to any monoclonal antibody bound to proteins containing the VRXQ motif. Unattached reporter antibody is then washed out. Peroxidase substrates are then added to the dish and the amount of color developed in a given time period is a measurement of the amount of protein containing the VRXQ motif present in a given volume of patient sample when compared against a standard curve to detect and quantitate the protein. Examples of other detectable reagents which can be used include, but are not limited to, luciferase and fluorescently or radioactively tagged secondary antibodies. Specific populations of immune cells or chimeric cells (e.g., hybridomas) that express antibodies to VRXQ epitopes on their cell surfaces and respond by degranulation or release of cellular contents such as histamines that can be detected functionally or preloaded radiolabeled metals such as chromium are also useful.

Embodiments of the invention can be used to detect alterations in and make comparisons between expression in of PS-1 variants in presumptive neurodegenerative disease, particularly neurodegenerative disease associated with head injury and AD, and more particularly chromosome 14 FAD. In a particularly preferred embodiment, probes and methods of the invention can be used to detect a reduction in the expression of PS-1 transcript encoding the VRSQ motif, shown by this invention to be a diagnostic marker for chromosome 14 FAD, since lowered levels are associated with chromosome 14 FAD. Preferred embodiments of the invention provide for comparisons between variants comprising the VRSQ region with those lacking it enabling the diagnosis of AD, particularly chromosome 14 FAD.

The methods of the invention to detect and quantitate PS-1 polynucleotide sequence, PS-1 expression levels and gene expression products, particularly the immunological methods and methods using oligonucleotides, can be used with bodily tissues and fluids from individuals. Preferred bodily tissues and fluids useful with the methods of the invention include, but are not limited to, blood cells, plasma, skin cells, and brain cells, particularly neuronal, glial, and astrocyte cells.

The following examples are provided for illustrative purposes only and are not intended to limit the invention.

### EXAMPLES

### Example 1

A novel splice variant of the PS-1 gene described by Sherrington et al. Nature 1995, 375:754-760, was isolated from a human cerebellar and a human fibroblast library. In this novel splice variant there is a deletion of four amino acids at codons 26-27 (VRSQ). This arises from alternative use of a 5' exon donor site in the exon3/intron 3 (-52 to 75 nt) boundary. The ...CAG/gta... boundary of the final Gln codon of exon 3 of the VRSQ motif provides a 5' exon AG donor site and GT intron consensus 5' boundary and use of this splice site results in the insertion of the 12-nts encoding the VRSQ motif. The upstream ...ACT/GTA... boundary of the Thr-Val codons provides the less preferred CT (AG preferred) 5' exonic boundary to the consensus GT 5' intronic boundary and splicing at this site would remove the VRSQ motif. Interestingly, in the PS-1 protein of Sherrington et al. Nature 1995, 375:754-760, this is the sole observed product and point mutations are interspersed elsewhere.

### Example 2

In the GABA receptor 4 subunit alternative splicing adds a VREQ motif (Bateson et al. J. Neurochem 1991, 56(4) 1437-40). A chicken genomic cDNA library was screened with chicken beta- 4' subunit cDNA at high stringency. Southern blot analysis, using cDNA sequence specific oligonucleotides as probes and subsequent restriction mapping allowed the identification of overlapping DNA fragments containing the coding regions of the beta-4 subunit gene. These fragments were subcloned into pBluescript and sequenced. Complete sequencing of one of the clones revealed the presence of 12 bp in the part encoding the intracellular loop (amino acid residues 335-338). Analysis of the beta-4 subunit gene reveals that the different transcripts encoding the two variants (absence or presence of 12bp loop) arise by the use of one of two 5'-donor splice sites (located in the intron immediately 3' of the 12 bp sequence).

### Example 3

The expression of two PS-1 mRNA transcripts, one containing (herein " PS-1-long ") and one lacking the VSRQ motif (herein "PS- 1-short"), in the brains of patients with early onset FAD was analyzed. In situ hybridization (ISH) was used to determine the qualitative and quantitative pattern of expression of PS-1 mRNA in the brains of early onset (presumptive chromosome 14-linked) FAD cases; comparisons with brains from patients with late onset AD and from normal individuals were made.

### In Situ Hybridization

PS-1 mRNA expression was examined in 4 neurologically normal control cases, 6 late onset AD cases and 3 early onset FAD cases. The late onset cases were thought to be of a sporadic nature as there was no evidence of family history and the mean age at death was 81.2 years (range: 79-84 years); they had a mean post mortem delay of 8.3 hours. The early onset FAD cases were presumed to be linked to chromosome 14 as they all had onset ages, family history, clinical presentations and histopathology typical of chromosome 14-linked FAD. For these the mean age at death was 45 years (range: 44-46 years) and the mean post mortem delay was 41.7 hours. All AD cases were diagnosed according to standard pathological criteria (Khachaturian, 1985, Archives of Neurology. 42:1097-1105). The controls had a mean age at death of 68.8 years (range: 57-85 years) and mean post mortem delay of 11.8 hours. The brain regions examined were the hippocampus, temporal cortex and frontal cortex (regions severely affected by AD pathology), the visual cortex (an area relatively unaffected, but which at the time of death may be in the early stages of the disease process) and the cerebellum (an area not affected by the classic pathology associated with AD and with no clinical involvement).

Three different oligoprobes were chosen and synthesized (Figure 4): one to detect PS-1-long, one to PS-1-short and one that recognizes both transcripts, PS-1-both, These probes are not predicted to detect the transcripts of presenilin-2, a closely related gene on chromosome 1 (Rogaev, et al., 1995, Nature 376:775-78).

The ISH methodology is well known in the art and has been described in detail elsewhere (Najlerahim et al., 1990, FEBS Letters 7:317-333). For the ISH analyses 10(m cryostat tissue sections were used. Probes were labelled at their 3' end with ³⁵S-dATP using the NEN DuPont 3' end labelling system. Hybridization and wash temperatures for the various probes are given in Figure 4. Hybridized sections were apposed to tritium-sensitive film for the generation of autoradiographs. Hybridization with the PS-1 probes in the sense orientation on adjacent sections were used to control for non-specific background. The signal on autoradiographic film was quantified using an image analyzer (Seescan®). A representative area over most of a tissue section was measured: for example, in the hippocampus the different subfields were not separately quantified. The background signal (sense strand hybridization) was subtracted from the antisense signal. Statistical analysis of the data was performed using the well known two-tailed Student's t-test.

### Northern Analysis

Northern analysis was carried out with the PS-1-both probe on a Northern blot (Clontech®, catalogue number: 7750-1) containing polyA+ mRNA from a number of different human brain regions. The probe was 3' end labelled with 32P-dATP using terminal transferase and hybridized under standard conditions (Clontech®, data sheet).

### Diagnostic Methods and Reagents for FAD

*In situ* hybridization using all three probes revealed that PS-1 mRNA was present in all of the brain regions examined. Hybridization with a sense strand control probe gave a very low background signal. In the cerebral cortex (three regions) a signal was detected in both the grey and white matter, often with a similar intensity. A diffuse rather than laminar pattern was observed in grey matter and in the hippocampus the different subfields were not readily delineated (although the dentate gyrus was sometimes visible). In the cerebellum, the granule cell layer contained the most labelling. These data are consistent with PS-1 mRNA expression in both neurons and glia.

Northern analysis confirmed that the PS-1-both oligoprobe detected a major transcript in human brain of the correct size for PS-1 mRNA (in accordance with the sequence data of Sherrington et al, 1995, Nature 375:754-760). A major band of approximately 3.4 kb was detected in all brain regions examined, indicating a wide distribution in brain for PS-1 mRNA. The observation of PS-1 mRNA in corpus callosum is consistent with the interpretation from our ISH data that PS-1 is expressed in glia.

A similar anatomical pattern was seen by ISH, in each region, for both PS-1-long and PS-1-short transcripts. Nevertheless there appeared to be differences between the transcripts in their levels of expression according to brain region; for example PS-1-short was relatively less abundant in the cerebellum (Figure 5).

The hybridization pattern was similar for the controls, sporadic AD and FAD cases. Quantification of the autoradiographic film revealed a statistically significant reduction in the amount of PS-1-long mRNA in FAD hippocampus and frontal cortex compared with the sporadic AD cases (Figure 5; p = 0.003 and p = 0.014 respectively). In the cerebellum there was no significant difference between the controls, sporadic AD and FAD cases. The reduction in PS-1-long appears to be specific because there was no change in the level of expression of PS-1-short mRNA in any brain region investigated between the three different groups (Figure 5), which indicates reasonable data consistency.

SEQUENCE LISTING

(1) GENERAL INFORMATION:
(i) APPLICANT: University of South Florida, Washington University and
SmithKline Beecham Corporation

(ii) TITLE OF INVENTION: A Novel Diagnostic Marker for Splicing
Variants of Genes Associated with Neurological Function

(iii) NUMBER OF SEQUENCES: 5
(iv) CORRESPONDENCE ADDRESS:
(A) ADDRESSEE: SmithKline Beecham Corporation
(B) STREET: 709 Swedeland Road, P.O. Box 1539
(C) CITY: King of Prussia
(D) STATE: PA
(E) COUNTRY: USA
(F) ZIP: 19406-0939
(v) COMPUTER READABLE FORM:
(A) MEDIUM TYPE: DISKETTE, 3.5 INCH, 1.44 Mb
STORAGE
(B) COMPUTER: IBM 486
(C) OPERATING SYSTEM: WINDOWS FOR WORKGROUPS
(D) SOFTWARE: WORDPERFECT 5.1
(vi) CURRENT APPLICATION DATA:
(A) APPLICATION NUMBER: not yet assigned
(B) FILING DATE: Herewith
(C) CLASSIFICATION:
(vii) PRIOR APPLICATION DATA:
(A) APPLICATION NUMBER:60/012,077
(B) FILING DATE:February 22, 1996
(viii) ATTORNEY/AGENT INFORMATION:
(A) NAME: William T. Han
(B) REGISTRATION NUMBER: 34,344
(C) REFERENCE/DOCKET NUMBER: ATG50003
(ix) TELECOMMUNICATION INFORMATION:
(A) TELEPHONE: 610-270-5024
(B) TELEFAX: 610-270-5090
(2) INFORMATION FOR SEQ ID NO: 1:
(i) SEQUENCE CHARACTERISTICS:
(A) LENGTH: 1914

(xi) SEQUENCE DESCRIPTION: SEQ ID NO: 1:
CCGTACGTAG CCGCGGCGGC AGCGGGGCGG CGGGGAAGCG TATGCATACA 50
AATTTATTAG CATGCAGACT GGGAGAACCA CAAGACCTAA TCTGGGAGCC 100
TGCAAGTGAC AACAGCCTTT GCGGTCCTTA GACAGCTTGG CCTGGAGGAG 150
AACACATGAA AGAAAGAACC TCAAGAGGCT TTGTTTTCTG TGAAACAGTA 200
TTTCTATACA GTTGCTCCAA TGACAGAGTT ACCTGCACCG TTGTCCTACT 250
TCCAGAATGC ACAGATGTCT GAGGACAACC ACCTGAGCAA TACTAATGAC 300
AATAGAGAAC GGCAGGAGCA CAACGACAGA CGGAGCCTTG GCCACCCTGA 350
GCCATTATCT AATGGACGAC CCCAGGGTAA CTCCCGGCAG GTGGTGGAGC 400
AAGATGAGGA AGAAGATGAG GAGCTGACAT TGAAATATGG CGCCAAGCAT 450
GTGATCATGC TCTTTGTCCC TGTGACTCTC TGCATGGTGG TGGTCGTGGC 500
TACCATTAAG TCAGTCAGCT TTTATACCCG GAAGGATGGG CAGCTAATCT 550
ATACCCCATT CACAGAAGAT ACCGAGACTG TGGGCCAGAG AGCCCTGCAC 600
TCAATTCTGA ATGCTGCCAT CATGATCAGT GTCATTGTTG TCATGACTAT 650
CCTCCTGGTG GTTCTGTATA AATACAGGTG CTATAAGGTC ATCCATGCCT 700
GGCTTATTAT ATCATCTCTA TTGTTGCTGT TCTTTTTTTC ATTCATTTAC 750
TTGGGGGAAG TGTTTAAAAC CTATAACGTT GCTGTGGACT ACATTACTGT 800
TGCACTCCTG ATCTGGAATT TTGGTGTGGT GGGAATGATT TCCATTCACT 850
GGAAAGGTCC ACTTCGACTC CAGCAGGCAT ATCTCATTAT GATTAGTGCC 900
CTCATGGCCC TGGTGTTTAT CAAGTACCTC CCTGAATGGA CTGCGTGGCT 950
CATCTTGGCT GTGATTTCAG TATATGATTT AGTGGCTGTT TTGTGTCCGA 1000
AAGGTCCACT TCGTATGCTG GTTGAAACAG CTCAGGAGAG AGATGAAACG 1050
CTTTTTCCAG CTCTCATTTA CTCCTCAACA ATGGTGTGGT TGGTGAATAT 1100
GGCAGAAGGA GACCCGGAAG CTCAAAGGAG AGTATCCAAA AATTCCAAGT 1150
ATAATGCAGA AAGCACAGAA AGGGAGTCAC AAGACACTGT TGCAGAGAAT 1200
GATGATGGCG GGTTCAGTGA GGAATGGGAA GCCCAGAGGG ACAGTCATCT 1250
AGGGCCTCAT CGCTCTACAC CTGAGTCACG AGCTGCTGTC CAGGAACTTT 1300
CCAGCAGTAT CCTCGCTGGT GAAGACCCAG AGGAAAGGGG AGTAAAACTT 1350
GGATTGGGAG ATTTCATTTT CTACAGTGTT CTGGTTGGTA AAGCCTCAGC 1400
AACAGCCAGT GGAGACTGGA ACACAACCAT AGCCTGTTTC GTAGCCATAT 1450
TAATTGGTTT GTGCCTTACA TTATTACTCC TTGCCATTTT CAAGAAAGCA 1500
TTGCCAGCTC TTCCAATCTC CATCACCTTT GGGCTTGTTT TCTACTTTGC 1550
CACAGATTAT CTTGTACAGC CTTTTATGGA CCAATTAGCA TTCCATCAAT 1600
TTTATATCTA GCATATTTGC GGTTAGAATC CCATGGATGT TTCTTCTTTG 1650
ACTATAACAA AATCTGGGGA GGACAAAGGT GRTTTTCCTG TGTCCCACAT 1700
CTAACAAAGT CAAGATTCCC GKCTGGACTT TTGCAGCTTC CTKCCAAGTC 1750
TTCCTGACCA CCTTGCACTW TTGGACTTTG GARGGAGGTG CCTAKAGAAA 1800
ACGRTTTTGA MCATACTTCA TCGCAGTGGA CTGTGTCCCT CGGTGCAGAA 1850
ACTACCAGAT TTGAGGGACG AGGTCAAGGA GATATGATAG GCCCGGAAGT 1900
TGCTGTGCCC ATCA 1914

1
(2) INFORMATION FOR SEQ ID NO: 2:
(i) SEQUENCE CHARACTERISTICS:
(A) LENGTH: 463
(B) TYPE: Amino Acid
(D) TOPOLOGY: Linear
(xi) SEQUENCE DESCRIPTION: SEQ ID NO: 2:
MET THR GLU LEU PRO ALA PRO LEU SER TYR PHE GLN ASN ALA GLN
1               5                   10                  15

MET SER GLU ASP ASN HIS LEU SER ASN THR ASN ASP ASN ARG GLU
                20                  25                  30

ARG GLN GLU HIS ASN ASP ARG ARG SER LEU GLY HIS PRO GLU PRO
                35                  40                  45

LEU SER ASN GLY ARG PRO GLN GLY ASN SER ARG GLN VAL VAL GLU
                50                  55                  60

GLN ASP GLU GLU GLU ASP GLU GLU LEU THR LEU LYS TYR GLY ALA
                65                  70                  75

LYS HIS VAL ILE MET LEU PHE VAL PRO VAL THR LEU CYS MET VAL
                80                  85                  90

VAL VAL VAL ALA THR ILE LYS SER VAL SER PHE TYR THR ARG LYS
                95                  100                 105

ASP GLY GLN LEU ILE TYR THR PRO PHE THR GLU ASP THR GLU THR
                110                 115                 120

VAL GLY GLN ARG ALA LEU HIS SER ILE LEU ASN ALA ALA ILE MET
                125                 130                 135

ILE SER VAL ILE VAL VAL MET THR ILE LEU LEU VAL VAL LEU TYR
                140                 145                 150

LYS TYR ARG CYS TYR LYS VAL ILE HIS ALA TRP LEU ILE ILE SER
                155                 160                 165

SER LEU LEU LEU LEU PHE GLU GLU SER PHE ILE TYR LEU GLY GLU
                170                 175                 180

VAL PHE LYS THR TYR ASN VAL ALA VAL ASP TYR ILE THR VAL ALA
                185                 190                 195

LEU LEU ILE TRP ASN PHE GLY VAL VAL GLY MET ILE SER ILE HIS
                200                 205                 210

TRP LYS GLY PRO LEU ARG LEU GLN GLN ALA TYR LEU ILE MET ILE
                215                 220                 225

SER ALA LEU MET ALA LEU VAL PHE ILE LYS TYR LEU PRO GLU TRP
                230                 235                 240

THR ALA TRP LEU ILE LEU ALA VAL ILE SER VAL TYR ASP LEU VAL
                245                 250                 255

ALA VAL LEU CYS PRO LYS GLY PRO LEU ARG MET LEU VAL GLU THR
                260                 265                 270

ALA GLN GLU ARG ASP GLU THR LEU PHE PRO ALA LEU ILE TYR SER
                275                 280                 285

SER THR MET VAL TRP LEU VAL ASN MET ALA GLU GLY ASP PRO GLU
                290                 295                 300

ALA GLN ARG ARG VAL SER LYS ASN SER LYS TYR ASN ALA GLU SER
                305                 310                 315

THR GLU ARG GLU SER GLN ASP THR VAL ALA GLU ASN ASP ASP GLY
                320                 325                 330

GLY PHE SER GLU GLU TRP GLU ALA GLN ARG ASP SER HIS LEU GLY
                335                 340                 345

PRO HIS ARG SER THR PRO GLU SER ARG ALA ALA VAL GLN GLU LEU
                350                 355                 360

SER SER SER ILE LEU ALA GLY GLU ASP PRO GLU GLU ARG GLY VAL
                365                 370                 375

LYS LEU GLY LEU GLY ASP PHE ILE PHE TYR SER VAL LEU VAL GLY
                380                 385                 390

LYS ALA SER ALA THR ALA SER GLY ASP TRP ASN THR THR ILE ALA
                395                 400                 405

CYS PHE VAL ALA ILE LEU ILE GLY LEU CYS LEU THR LEU LEU LEU
                410                 415                 420

LEU ALA ILE PHE LYS LYS ALA LEU PRO ALA LEU PRO ILE SER ILE
                425                 430                 435

THR PHE GLY LEU VAL PHE TYR PHE ALA THR ASP TYR LEU VAL GLN
                440                 445                 450

PRO PHE MET ASP GLN LEU ALA PHE HIS GLN PHE TYR ILE
                455                 460

(2) INFORMATION FOR SEQ ID NO: 3:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO: 3:

GCACTCAATT CTGAATGCTG CCATCATGAT 30

(2) INFORMATION FOR SEQ ID NO: 4:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO: 4:

AGCAATACTG TACGTAGCCA GAATGACAAT 30

(2) INFORMATION FOR SEQ ID NO: 5:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO: 5:

CACCTGAGCA ATACWATGAC AATAGAGAA 29

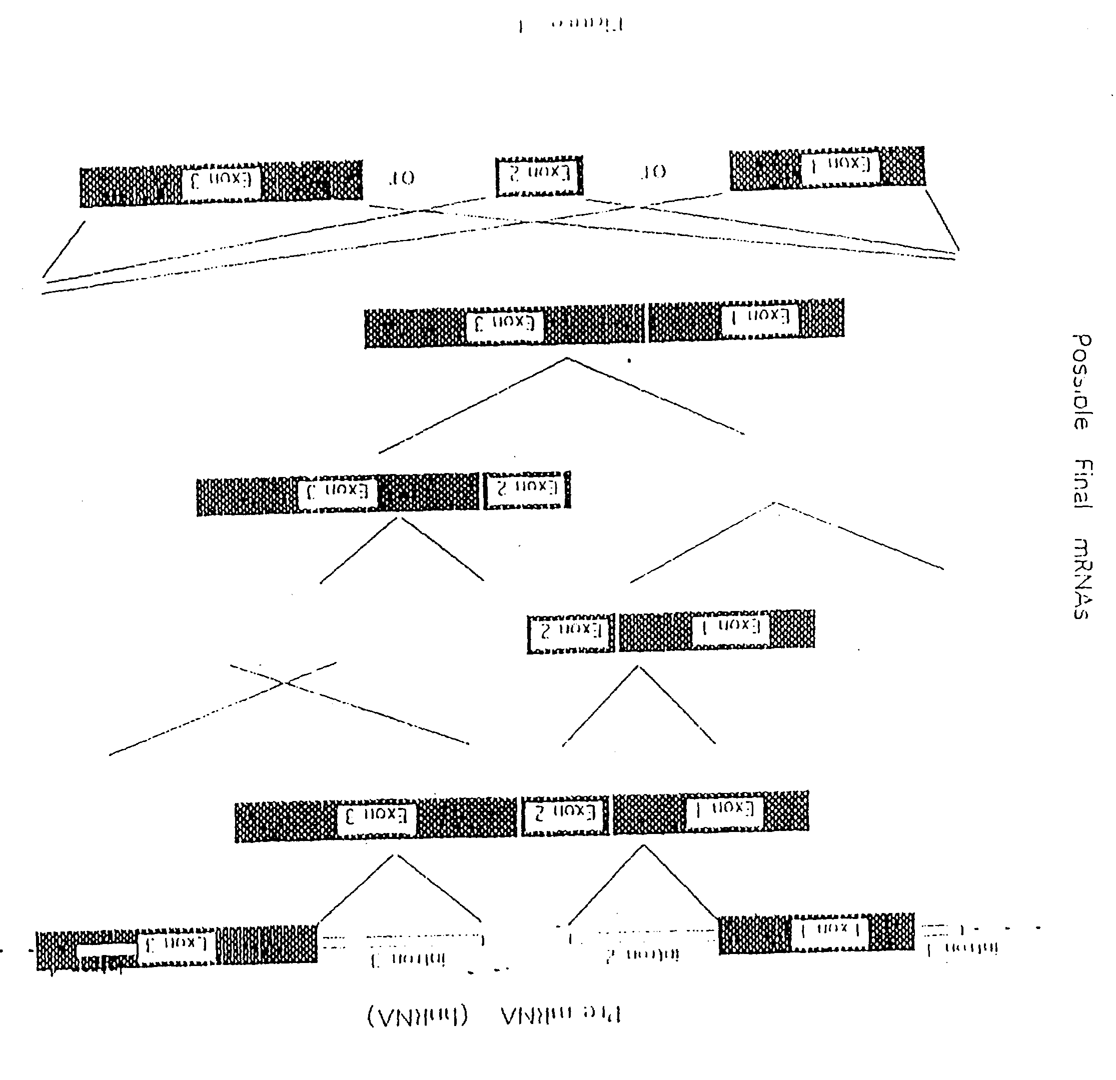
Pre mRNA (hnRNA)

Intron 1
Exon 1
Intron 2
Intron 3
Exon 3


Exon 1
Exon 2
Exon 3


Exon 1
Exon 2

Exon 2
Exon 3


Exon 1
Exon 3


Exon 1
or
Exon 2
or
Exon 3

Possible Final mRNAs

Figure 1

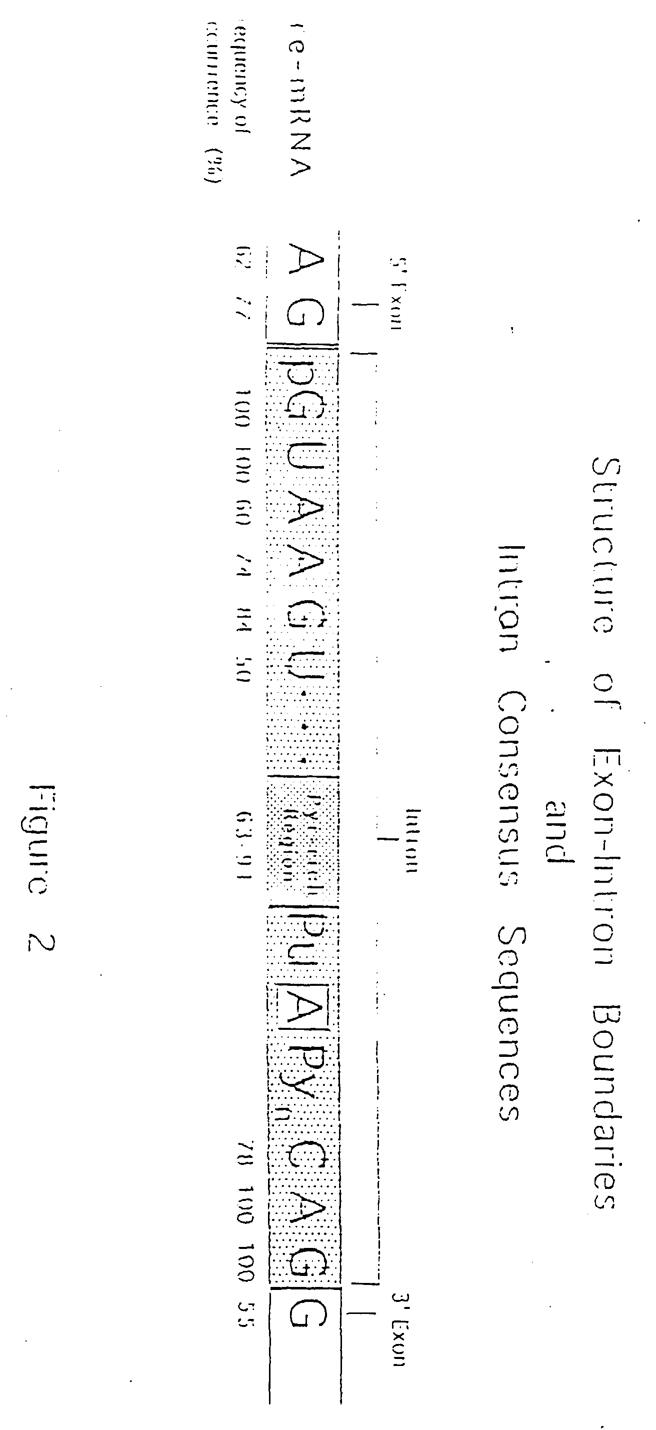
Structure of Exon-Intron Boundaries
and
Intron Consensus Sequences

5' Exon
Intron
3' Exon

re-mRNA | A G | pG U A A G U • • | Py-rich Region | Pu A Py n C A G | G

equency of
ccurrence (%) | 62 77 | 100 100 60 74 84 50 | 63-91 | 78 100 100 | 55


Figure 2

FIGURE 1A

11 20 29 38 47 56
5' CCG TAC GTA GCC GCG GCG GCA GCG GGG CGG CGG GGA AGC GTA TGC ATA CAA ATT
--- --- --- --- --- --- --- --- --- --- --- --- --- --- --- --- --- ---

65 74 83 92 101 110
TAT TAG CAT GCA GAC TGG GAG AAC CAC AAG ACC TAA TCT GGG AGC CTG CAA GTG
--- --- --- --- --- --- --- --- --- --- --- --- --- --- --- --- --- ---

119 128 137 146 155 164
ACA ACA GCC TTT GCG GTC CTT AGA CAG CTT GGC CTG GAG GAG AAC ACA TGA AAG
--- --- --- --- --- --- --- --- --- --- --- --- --- --- --- --- --- ---

173 182 191 200 209 218
AAA GAA CCT CAA GAG GCT TTG TTT TCT GTG AAA CAG TAT TTC TAT ACA GTT GCT
--- --- --- --- --- --- --- --- --- --- --- --- --- --- --- --- --- ---

227 236 245 254 263 272
CCA ATG ACA GAG TTA CCT GCA CCG TTG TCC TAC TTC CAG AAT GCA CAG ATG TCT
--- --- --- --- --- --- --- --- --- --- --- --- --- --- --- --- --- ---
M T E L P A P L S Y F Q N A Q M S

281 290 299 308 317 326
GAG GAC AAC CAC CTG AGC AAT ACT AAT GAC AAT AGA GAA CGG CAG GAG CAC AAC
--- --- --- --- --- --- --- --- --- --- --- --- --- --- --- --- --- ---
E D N H L S N T N D N R E R Q E H N

335 344 353 362 371 380
GAC AGA CGG AGC CTT GGC CAC CCT GAG CCA TTA TCT AAT GGA CGA CCC CAG GGT
--- --- --- --- --- --- --- --- --- --- --- --- --- --- --- --- --- ---
D R R S L G H P E P L S N G R P Q G

389 398 407 416 425 434
AAC TCC CGG CAG GTG GTG GAG CAA GAT GAG GAA GAA GAT GAG GAG CTG ACA TTG
--- --- --- --- --- --- --- --- --- --- --- --- --- --- --- --- --- ---
N S R Q V V E Q D E E E D E E L T L

443 452 461 470 479 488
AAA TAT GGC GCC AAG CAT GTG ATC ATG CTC TTT GTC CCT GTG ACT CTC TGC ATG
--- --- --- --- --- --- --- --- --- --- --- --- --- --- --- --- --- ---
K Y G A K H V I M L F V P V T L C M

497 506 515 524 533 542
GTG GTG GTC GTG GCT ACC ATT AAG TCA GTC AGC TTT TAT ACC CGG AAG GAT GGG
--- --- --- --- --- --- --- --- --- --- --- --- --- --- --- --- --- ---
V V V V A T I K S V S F Y T R K D G

FIGURE 1B

551 560 569 578 587 596
CAG CTA ATC TAT ACC CCA TTC ACA GAA GAT ACC GAG ACT GTG GGC CAG AGA GCC
--- --- --- --- --- --- --- --- --- --- --- --- --- --- --- --- --- ---
Q L I Y T P F T E D T E T V G Q R A

605 614 623 632 641 650
CTG CAC TCA ATT CTG AAT GCT GCC ATC ATG ATC AGT GTC ATT GTT GTC ATG ACT
--- --- --- --- --- --- --- --- --- --- --- --- --- --- --- --- --- ---
L H S I L N A A I M I S V I V V M T

659 668 677 686 695 704
ATC CTC CTG GTG GTT CTG TAT AAA TAC AGG TGC TAT AAG GTC ATC CAT GCC TGG
--- --- --- --- --- --- --- --- --- --- --- --- --- --- --- --- --- ---
I L L V V L Y K Y R C Y K V I H A W

713 722 731 740 749 758
CTT ATT ATA TCA TCT CTA TTG TTG CTG TTC TTT TTT TCA TTC ATT TAC TTG GGG
--- --- --- --- --- --- --- --- --- --- --- --- --- --- --- --- --- ---
L I I S S L L L L F E F S F I Y L G

767 776 785 794 803 812
GAA GTG TTT AAA ACC TAT AAC GTT GCT GTG GAC TAC ATT ACT GTT GCA CTC CTG
--- --- --- --- --- --- --- --- --- --- --- --- --- --- --- --- --- ---
E V F K T Y N V A V D Y I T V A L L

821 830 839 848 857 866
ATC TGG AAT TTT GGT GTG GTG GGA ATG ATT TCC ATT CAC TGG AAA GGT CCA CTT
--- --- --- --- --- --- --- --- --- --- --- --- --- --- --- --- --- ---
I W N F G V V G M I S I H W K G P L

875 884 893 902 911 920
CGA CTC CAG CAG GCA TAT CTC ATT ATG ATT AGT GCC CTC ATG GCC CTG GTG TTT
--- --- --- --- --- --- --- --- --- --- --- --- --- --- --- --- --- ---
R L Q Q A Y L I M I S A L M A L V F

929 938 947 956 965 974
ATC AAG TAC CTC CCT GAA TGG ACT GCG TGG CTC ATC TTG GCT GTG ATT TCA GTA
--- --- --- --- --- --- --- --- --- --- --- --- --- --- --- --- --- ---
I K Y L P E W T A W L I L A V I S V

983 992 1001 1010 1019 1028
TAT GAT TTA GTG GCT GTT TTG TGT CCG AAA GGT CCA CTT CGT ATG CTG GTT GAA
--- --- --- --- --- --- --- --- --- --- --- --- --- --- --- --- --- ---
Y D L V A V L C P K G P L R M L V E

1037 1046 1055 1064 1073 1082
ACA GCT CAG GAG AGA GAT GAA ACG CTT TTT CCA GCT CTC ATT TAC TCC TCA ACA
--- --- --- --- --- --- --- --- --- --- --- --- --- --- --- --- --- ---
T A Q E R D E T L F P A L I Y S S T

1091 1100 1109 1118 1127 1136
ATG GTG TGG TTG GTG AAT ATG GCA GAA GGA GAC CCG GAA GCT CAA AGG AGA GTA

FIGURE 1C

--- --- --- --- --- --- - --- --- --- --- --- --- --- --- - --- ---

M V W L V N M A E G D P E A Q R R V

1145 1154 1163 1172 1181 1190
TCC AAA AAT TCC AAG TAT AAT GCA GAA AGC ACA GAA AGG GAG TCA CAA GAC ACT
--- --- --- --- --- --- --- --- --- --- --- --- --- --- --- --- --- ---
S K N S K Y N A E S T E R E S Q D T

1199 1208 1217 1226 1235 1244
GTT GCA GAG AAT GAT GAT GGC GGG TTC AGT GAG GAA TGG GAA GCC CAG AGG GAC
--- --- --- --- --- --- --- --- --- --- --- --- --- --- --- --- --- ---
V A E N D D G G F S E E W E A Q R D

1253 1262 1271 1280 1289 1298
AGT CAT CTA GGG CCT CAT CGC TCT ACA CCT GAG TCA CGA GCT GCT GTC CAG GAA
--- --- --- --- --- --- --- --- --- --- --- --- --- --- --- --- --- ---
S H L G P H R S T P E S R A A V Q E

1307 1316 1325 1334 1343 1352
CTT TCC AGC AGT ATC CTC GCT GGT GAA GAC CCA GAG GAA AGG GGA GTA AAA CTT
--- --- --- --- --- --- --- --- --- --- --- --- --- --- --- --- --- ---
L S S S I L A G E D P E E R G V K L

1361 1370 1379 1388 1397 1406
GGA TTG GGA GAT TTC ATT TTC TAC AGT GTT CTG GTT GGT AAA GCC TCA GCA ACA
--- --- --- --- --- --- --- --- --- --- --- --- --- --- --- --- --- ---
G L G D F I F Y S V L V G K A S A T

1415 1424 1433 1442 1451 1460
GCC AGT GGA GAC TGG AAC ACA ACC ATA GCC TGT TTC GTA GCC ATA TTA ATT GGT
--- --- --- --- --- --- --- --- --- --- --- --- --- --- --- --- --- ---
A S G D W N T T I A C F V A I L I G

1469 1478 1487 1496 1505 1514
TTG TGC CTT ACA TTA TTA CTC CTT GCC ATT TTC AAG AAA GCA TTG CCA GCT CTT
--- --- --- --- --- --- --- --- --- --- --- --- --- --- --- --- --- ---
L C L T L L L L A I F K K A L P A L

1523 1532 1541 1550 1559 1568
CCA ATC TCC ATC ACC TTT GGG CTT GTT TTC TAC TTT GCC ACA GAT TAT CTT GTA
--- --- --- --- --- --- --- --- --- --- --- --- --- --- --- --- --- ---
P I S I T F G L V F Y F A T D Y L V

1577 1586 1595 1604 1613 1622
CAG CCT TTT ATG GAC CAA TTA GCA TTC CAT CAA TTT TAT ATC TAG CAT ATT TGC
--- --- --- --- --- --- --- --- --- --- --- --- --- --- --- --- --- ---
Q P F M D Q L A F H Q F Y I *.

1631 1640 1649 1658 1667 1676
GGT TAG AAT CCC ATG GAT GTT TCT TCT TTG ACT ATA ACA AAA TCT GGG GAG GAC
--- --- --- --- --- --- --- --- --- --- --- --- --- --- --- --- --- ---

FIGURE 1D

1685 1694 1703 1712 1721 1730
AAA GGT GRT TTT CCT GTG TCC CAC ATC TAA CAA AGT CAA GAT TCC CGK CTG GAC
--- --- --- --- --- --- --- --- --- --- --- --- --- --- --- --- --- ---

1739 1748 1757 1766 1775 1784
TTT TGC AGC TTC CTK CCA AGT CTT CCT GAC CAC CTT GCA CTW TTG GAC TTT GGA
--- --- --- --- --- --- --- --- --- --- --- --- --- --- --- --- --- ---

1793 1802 1811 1820 1829 1838
RGG AGG TGC CTA KAG AAA ACG RTT TTG AMC ATA CTT CAT CGC AGT GGA CTG TGT
--- --- --- --- --- --- --- --- --- --- --- --- --- --- --- --- --- ---

1847 1856 1865 1874 1883 1892
CCC TCG GTG CAG AAA CTA CCA GAT TTG AGG GAC GAG GTC AAG GAG ATA TGA TAG
--- --- --- --- --- --- --- --- --- --- --- --- --- --- --- --- --- ---

1901 1910
GCC CGG AAG TTG CTG TGC CCA TCA 3'
--- --- --- --- --- --- --- ---

Figure 4

PS-1 Oligonucleotide Probes

Probe | Sense Sequence | Bases * | Ti °C | Tw °C

PS-1-both | 5'-GCACTCAATTCTGAATGCTGCCATCATGAT-3' | 638-667 | 24 | 50
SEQ ID NO: 3

PS-1-long | 5-'AGCAATACTGTACGTAGCCAGAATGACAAT-3' | 315-344 | 23 | 49
SEQ ID NO: 4

PS-1-short | 5'-CACCTGAGCAATACT/AATGACAATAGAGAA-3' | 309-323 and 336-350 | 22 | 47
SEQ ID NO: 5

*Refers to EMBL and GenBank entry HUMS182R (accession number: L42110); Sherrington et al
1995, Nature 375:754-760. Ti represents the hybridization temperature (incubation) and Tw
represents the wash temperature. The underlined bases code for the amino acids V, R, S and Q.

Figure 5.

Quantification of the ISH signal for PS-1-long and PS-1-short mRNAs in human brain.

Brain region | Case | PS-1-long (n) | PS-1-short (n)

Hippocampus | Control | 0.025 ± 0.014 (2) | 0.023 (1)
| AD | 0.035 ± 0.007 (3) | 0.026 ± 0.01 (3)
| FAD | 0.008 ± 0.001 (3)* | 0.030 ± 0.004 (3)
Frontal cortex | AD | 0.024 ± 0.005 (3) | 0.042 ± 0.014 (3)
| FAD | 0.012 ± 0.0 (3)** | 0.022 ± 0.011 (3)
Cerebellum | Control | 0.036 (1) | 0.013 (1)
| AD | 0.024 ± 0.007 (3) | 0.019 ± 0.006 (3)
| FAD | 0.012 ± 0.002 (2) | 0.014 ± 0.005 (2)
Temporal cortex | FAD | 0.014 ± 0.009 (3) | 0.015 ± 0.01 (3)
Visual Cortex | FAD | 0.016 ± 0.007 (3) | 0.032 ± 0.001 (3)

values represent means ± s.d.; units are arbitrary (machine grey levels). *FAD vs AD p =
0.003; **FAD vs AD p = 0.014; Student's t-test.

## Claims

1. A method of identifying an individual susceptible to a neurological disease comprising:
providing a sample of genetic material from an individual susceptible to a neurological disease; and
detecting the presence of an alternative splice site comprising the sequence VRXQ, wherein V is valine, R is arginine, X is any hydrophobic amino acid residue and Q is glutamine, in a polyadenylated messenger RNA transcript or protein encoded therefrom in the sample of genetic material.

2. The method of claim 1 wherein the sequence VRXQ is detected using selected oligonucleotide probes comprising anticodon sequences associated with the sequence VRXQ having degenerate positions at the third base position.

3. The method of claim 2 further comprising associating said oligonucleotides with codon sequences and detecting cDNA.

4. The method of claim 1 wherein the sequence VRXQ is detected using an antibody against a polypeptide comprising the sequence VRXQ.

5. The method of claim 1 wherein the neurological disease comprises Alzheimer's Disease and the mRNA or protein is encoded by the presenilin 1 gene.

6. The method of claim 5 therein the sequence comprises a 4 amino acid insertion between codons 26 and 27 of the gene and the sequence VRSQ.

7. The method of claim 1 wherein the mRNA or protein is encoded by the gamma-Aminobutyric acid A receptor gene and the sequence comprises VREQ.

8. The method of claim 1 wherein the mRNA or protein is encoded by the tyrosine hydroxylase gene and the sequence comprises VRGQ.

9. A method for diagnosing a neurological disease comprising determining the levels of polyadenylated messenger RNA transcripts or proteins encoded therefrom comprising the sequence VRXQ wherein V is valine, R is arginine, X is any hydrophobic amino acid residue and Q is glutamine, in a sample of genetic material and comparing these levels with established controls.

10. The method of claim 9 wherein the neurological disease comprises Alzheimer's Disease and the mRNA or protein is encodes by the presenilin 1 gene.

11. The method of claim 10 wherein the sequence comprises a 4 amino acid insertion between codons 26 and 27 of the gene and the sequence VRSQ.

12. The method of claim 9 wherein the mRNA or protein is encoded by the gamma-Aminobutyric acid A receptor gene and the sequence comprises VREQ.

13. The method of claim 9 wherein the mRNA or protein is encoded by the tyrosine hydroxylase gene and the sequence comprises VRGQ.
